# EUROPEAN PATENT SPECIFICATION

(11) **EP 0 935 591 B1**
(45) Date of publication and mention of the grant of the patent: **14.09.2005**
(21) Application number: 97911629.0
(22) Date of filing: 02.10.1997
(51) Int. Cl.: C07C 1/20, C07C 2/66, B01J 21/00, B01J 21/02, B01J 21/10, B01J 29/06

(54) **SELECTIVE PARA-XYLENE PRODUCTION BY TOLUENE METHYLATION**
SELEKTIVE HERSTELLUNG VON P-XYLOL DURCH METHYLIERUNG VON TOLUOL
PRODUCTION SELECTIVE DE PARA-XYLENE PAR METHYLATION DE TOLUENE

(30) Priority: 02.10.1996 US 725277
(43) Date of publication of application: 18.08.1999
(73) Proprietor: EXXONMOBIL OIL CORPORATION, Fairfax, VA 22037 (US)
(72) Inventor: BROWN, Stephen, Harold, Princeton, NJ 08540 (US); MATHIAS, Mark, Fischer, Turnersville, NJ 08012 (US); OLSON, David, Harold, Pennnington, NJ 08534 (US); WARE, Robert, Adams, Wyndmoor, PA 19038 (US)
(74) Representative: Kador & Partner
(86) International application number: PCT/US1997/018079
(87) International publication number: WO 1998/014415

(56) References cited:
- US-A- 4 380 685
- US-A- 4 491 678

## Description

There is provided a process for the selective production of para-xylene by catalytic methylation of toluene in the presence of a solid catalyst. There is also provided a method for preparing a catalyst which is particularly suited for this reaction.

Of the xylene isomers, para-xylene is of particular value since it is useful in the manufacture of terephthalic acid which is an intermediate in the manufacture of synthetic fibers. Equilibrium mixtures of xylene isomers either alone or in further admixture with ethylbenzene generally contain only about 24 wt% para-xylene and separation of p-xylene from such mixtures has typically required superfractionation and multistage refrigeration steps. Such processes have involved high operation costs and resulted in only limited yields. There is therefore a continuing need to provide processes which are highly selective for the production of p-xylene.

One known method for producing xylenes involves the alkylation of toluene with methanol over a solid acid catalyst. Thus the alkylation of toluene with methanol over cation-exchanged zeolite Y has been described by Yashima et al. in the Journal of Catalysis 16, 273-280 (1970). These workers reported selective production of para-xylene over the approximate temperature range of 200 to 275°C, with the maximum yield of para-xylene in the mixture of xylenes, i.e. about 50% of the xylene product mixture, being observed at 225°C. Higher temperatures were reported to result in an increase in the yield of meta-xylene and a decrease in production of para and ortho-xylenes.

U.S. Patent Nos. 3,965,209 to Butter et al. and 4,067,920 to Keading teach processes for producing para-xylene in low conversion and high selectivity by reaction of toluene with methanol over a zeolite having a Constraint Index of 1-12, such as ZSM-5. In Butter et al the zeolite is steamed at a temperature of 250-1000°C for 0.5-100 hours to reduce the acid activity of the zeolite, as measured by its alpha activity, to less than 500 and preferably from in excess of zero to less than 20.

U.S. Patent No. 4,001,346 to Chu relates to a process for the selective production of para-xylene by methylation of toluene in the presence of a catalyst comprising a crystalline aluminosilicate zeolite which has undergone prior treatment to deposit a coating of between about 15 and about 75 wt% of coke thereon.

U.S. Patent No. 4,097,543 to Haag et al. relates to a process for the selective production of para-xylene (up to about 77%) by disproportionation of toluene in the presence of a crystalline aluminosilicate catalyst which has undergone precooking to deposit a coating of at least about 2 wt% coke thereon.

U.S. Patent No. 4,380,685 to Chu relates to a process for para-selective aromatics alkylation, including the methylation of toluene, over a zeolite, such as ZSM-5, which has a constraint index of 1-12 and which has been combined with phosphorus and a metal selected from iron and cobalt. Chu indicates that the catalyst can optionally be modified (without specifying the effect of the modification) by steaming at a temperature of 250-1000°C, preferably 400-700°C for 0.5-100 hours, preferably 1-24 hours.

U.S. Patent No. 4,554,394 to Forbus and Kaeding teach the use of phosphorus-treated zeolite catalysts for enhancing para-selectivity in aromatics conversion processes. U.S. Patent No. 4,623,633 to Young relates to the use of thermal shock calcinations of aluminosilicates to produce up to 66% para-xylene selectivity.

The use of phosphorus modified ZSM-5 fluid bed catalysts as additive catalysts to improve the olefin yield in fluid catalytic cracking (FCC) is described in U.S. Patent No. 5,389,232 to Adewuyi et al. and in U.S. Patent No. 5,472,594 to Tsang et al.

According to the invention, it has now been found that certain porous crystalline materials having specific and closely defined diffusion characteristics, such as can be obtained by unusually severe steaming of ZSM-5 containing an oxide modifier, exhibit improved selectivity for the alkylation of toluene with methanol such that the xylene product contains at least about 90% of the para-isomer at per-pass toluene conversions of at least about 15%.

In one aspect, the invention resides in process for the selective production of para-xylene which comprises reacting toluene with methanol under alkylation conditions in the presence of a catalyst comprising a porous crystalline material having a Diffusion Parameter for 2,2-dimethylbutane of 0.1-15 sec⁻¹ when measured at a temperature of 120°C and a 2,2-dimethylbutane pressure of 8 kPa (60 torr); and wherein said porous crystalline material having a Diffusion Parameter of greater than 15 sec⁻¹ has undergone prior treatment with steam at a temperature in excess of 1000 °C to adjust the Diffusion Parameter of said material to 0.1-15 sec⁻¹ and the steaming reduces the pore volume of the catalyst when measured by n-hexane adsorption at a temperature of 90 °C and a n-hexane pressure of 10 kPa (75 torr) to not less than 50 % of that of the catalyst prior to said steam treatment.

Preferably, the porous crystalline material has a Diffusion Parameter of 0.5-10 sec⁻¹.

Preferably, the catalyst contains at least one oxide modifier and more preferably at least one oxide modifier selected from oxides of elements of Groups IIA, IIIA, IVA, VA, VB, and VIA of the Periodic Table. Most preferably the oxide modifier is selected from oxides of boron, magnesium, calcium, lanthanum and most preferably phorphorus.

Preferably, the catalyst contains 0.05 to 20, more preferably 0.1 to 10 and most preferably 0.1 to 5 wt% of the oxide modifier based on elemental modifier.

Preferably, the catalyst has an alpha value less than 50 and preferably less than 10.

In a further aspect, the invention resides in a method for producing a catalyst for use in the selective production of para-xylene by reacting toluene with methanol, said method comprising the steps of:
(a) starting with a porous crystalline material having a Diffusion Parameter for 2,2-dimethylbutane in excess of 15 sec⁻¹ when measured at a temperature of 120 °C and a 2,2-dimethylbutane pressure of 8 kPa (60 torr); and
(b) contacting the material of step (a) with steam at a temperature of at least 1000 °C to reduce the Diffusion Parameter thereof for 2,2-dimethylbutane to 0.1-15 sec⁻¹ when measured at a temperature of 120 °C and a 2,2-dimethylbutane pressure of 8 kPa (60 torr), the micropore volume of the steamed material when measured by n-hexane adsorption at a temperature of 90 °C and a n-hexane pressure of 10 kPa (75 torr) being at least 50 % of the catalyst prior to said steam treatment.

Preferably, said porous crystalline material used in step (a) comprises an aluminosilicate zeolite having a silica to alumina molar ratio of at least 250.

The present invention provides a process for alkylating toluene with methanol to selectively produce p-xylene in high yield and with a high perpass conversion of toluene. The process employs a catalyst which comprises a porous crystalline material having a Diffusion Parameter for 2,2-dimethylbutane of 0.1-15 sec⁻¹, and preferably 0.5-10 sec⁻¹, when measured at a temperature of 120 °C and a 2,2-dimethylbutane pressure of 8 kPa (60 torr).

As used herein, the Diffusion Parameter of a particular porous crystalline material is defined as D/r²x10⁶, wherein D is the diffusion coefficient (cm²/sec) and r is the crystal radius (cm). The required diffusion parameters can be derived from sorption measurements provided the assumption is made that the plane sheet model describes the diffusion process. Thus for a given sorbate loading Q, the value Q/Q.., where Q.. is the equilibrium sorbate loading, is mathematically related to (Dt/r²)^{1/2} where t is the time (sec) required to reach the sorbate loading Q. Graphical solutions for the plane sheet model are given by J. Crank in "The Mathematics of Diffusion", Oxford University Press, Ely House, London, 1967.

The porous crystalline material employed in the process of the invention is preferably a medium-pore size aluminosilicate zeolite. Medium pore zeolites are generally defined as those having a pore size of about 5 to about 7 Angstroms, such that the zeolite freely sorbs molecules such as n-hexane, 3-methylpentane, benzene and p-xylene. Another common definition for medium pore zeolites involves the Constraint Index test which is described in U.S. Patent No. 4,016,218, which is incorporated herein by reference. In this case, medium pore zeolites have a Constraint Index of 1-12, as measured on the zeolite alone without the introduction of oxide modifiers and prior to any steaming to adjust the diffusivity of the catalyst. In addition to the medium-pore size aluminosilicate zeolites, other medium pore acidic metallosilicates, such as silicoaluminophosphates (SAPOs), can be used in the process of the invention.

Particular examples of suitable medium pore zeolites include ZSM-5, ZSM-11, ZSM-12, ZSM-22, ZSM-23, ZSM-35, ZSM-48, and MCM-22, with ZSM-5 and ZSM-11 being particularly preferred.

Zeolite ZSM-5 and the conventional preparation thereof are described in U.S. Patent No. 3,702,886, the disclosure of which is incorporated herein by reference. Zeolite ZSM-11 and the conventional preparation thereof are described in U.S. Patent NO. 3,709,979, the disclosure of which is incorporated herein by reference. Zeolite ZSM-12 and the conventional preparation thereof are described in U.S. Patent NO. 3,832,449, the disclosure of which is incorporated herein by reference. Zeolite ZSM-23 and the conventional preparation thereof are described in U.S. Patent No. 4,076,842, the disclosure of which is incorporated herein by reference. Zeolite ZSM-35 and the conventional preparation thereof are described in U.S. Patent No. 4,016,245, the disclosure of which is incorporated herein by reference. ZSM-48 and the conventional preparation thereof is taught by U.S. Patent No. 4,375,573, the disclosure of which is incorporated herein by reference. MCM-22 is disclosed in U.S. Patent Nos. 5,304,698 to Husain; 5,250,277 to Kresge et al.; 5,095,167 to Christensen; and 5,043,503 to Del Rossi et al., the disclosure of which patents are incorporated by reference.

Preferably, the zeolite employed in the process of the invention is ZSM-5 having a silica to alumina molar ratio of at least 250, as measured prior to any treatment of the zeolite to adjust its diffusivity.

The medium pore zeolites described above are preferred for the process of the invention since the size and shape of their pores favor the production of p-xylene over the xylene isomers. However, conventional forms of these zeolites have Diffusion Parameter values in excess of the 0.1-15 sec⁻¹ range required for the process of the invention. The required diffusivity for the present catalyst can be achieved by severely steaming the catalyst so as to effect a controlled reduction in the micropore volume of the catalyst to not less than 50%, and preferably 50-90%, of that of the unsteamed catalyst. Reduction in micropore volume is derived by measuring the n-hexane adsorption capacity of the catalyst, before and after steaming, at 90°C and 10 kPa (75 torr) n-hexane pressure.

Steaming the porous crystalline material is effected at a temperature in excess of 1000°C, preferably 1000 to 1050°C for 10 minutes to 10 hours, preferably from 30 minutes to 5 hours.

To effect the desired controlled reduction in diffusivity and micropore volume, it may be desirable to combine the porous crystalline material, prior to steaming, with at least one oxide modifier, preferably selected from oxides of the elements of Groups IIA, IIIA, IIIB, IVA, VA, VB and VIA of the Periodic Table (IUPAC version). Most preferably, said at least one oxide modifier is selected from oxides of boron, magnesium, calcium, lanthanum, and most preferably phosphorus. In some cases, it may be desirable to combine the porous crystalline material with more than one oxide modifier, for example a combination of phosphorus with calcium and/or magnesium, since in this way it may be possible to reduce the steaming severity needed to achieve a target diffusivity value. The total amount of oxide modifier present in the catalyst, as measured on an elemental basis, may be between 0.05 and 20 wt%, and preferably is between 0.1 and 10 wt%, based on the weight of the final catalyst.

Where the modifier includes phosphorus, incorporation of modifier in the catalyst of the invention is conveniently achieved by the methods described in U.S. Patent Nos. 4,356,338; 5,110,776; 5,231,064 and 5,348,643, the entire disclosures of which are incorporated herein by reference. Treatment with phosphorus containing compounds can readily be accomplished by contacting the porous crystalline material, either alone or in combination with a binder or matrix material, with a solution of an appropriate phosphorus compound, followed by dying and calcining to convert the phosphorus to its oxide form. Contact with the phosphorus-containing compound is generally conducted at a temperature of about 25°C and about 125°C for a time between about 15 minutes and about 20 hours. The concentration of the phosphorus in the contact mixture may be between about 0.01 and about 30 wt%.

After contacting with the phosphorus-containing compound, the porous crystalline material may be dried and calcined to convert the phosphorus to an oxide for. Calcination can be carried out in an inert atmosphere or in the presence of oxygen, for example, in air at a temperature of about 150 to 750°C, preferably about 300 to 500°C, for at least 1 hour, preferably 3-5 hours.

Similar techniques known in the art can be used to incorporate other modifying oxides into the catalyst of the invention.

Representative phosphorus-containing compounds which may be used to incorporate a phosphorus oxide modifier into the catalyst of the invention include derivatives of groups represented by PX₃, RPX₂, R₂PX, R₃P, X₃PO, (XO)₃PO, (XO)₃P, R₃P=O, R₃P=S, RPO₂, RPS₂, RP(O)(OX)₂, RP(S)(SX)₂, R₂P(O)OX, R₂P(S)SX, RP(OX)₂, RP(SX)₂, ROP(OX)₂, RSP(SX)₂, (RS)₂PSP(SR)₂, and (RO)₂POP(OR)₂, where R is an alkyl or aryl, such as phenyl radical, and X is hydrogen, R, or halide. These compounds include primary, RPH₂, secondary, R₂PH, and tertiary, R₃P, phosphines such as butyl phosphine, the tertiary phosphine oxides, R₃PO, such as tributyl phosphine oxide, the tertiary phpsphines sulfides, R₃PS, the primary, RP(O)(OX)₂, and secondary, R₂P(O)(OX), phosphonic acids such as benzene phosphonic acid, the corresponding sulfur derivatives such as RP(S)(SX)₂ and R₂P(S)(SX), the esters of the phosphonic acids such as dialkyl phosphonate, (RO)₂P(O)H, dialkyl alkyl phosphonates, (RO)₂P(O)R, and alkyl dialkylphosphinates, (RO)P(O)R₂; phosphinous acids, R₂POX, such as diethylphosphinous acid, primary, (RO)P(OX)₂, secondary, (RO)₂POX, and tertiary, (RO)₃P, phosphites and esters thereof such as the monopropyl ester, alkyl dialkylphosphinites, (RO)PR₂, and dialkyl alkylphosphinite, (RO)₂PR, esters. Corresponding sulfur derivatives may also be employed including (RS)₂P(S)H, (RS)₂P(S)R, (RS)P(S)R₂, R₂PSX, (RS)P(SX)₂, (RS)P(SX)₂, (RS)₂PSX, (RS)₃P, (RS)PR₂, and (RS)₂PR. Examples of phosphite esters include trimethylphosphite, triethylphosphite, diisopropylphosphite, butylphosphite, and pyrophosphates such as tetraethylpyrophosphite. The alkyl groups in the mentioned compounds preferably contain one to four carbon atoms.

Other suitable phosphorus-containing compounds include ammonium hydrogen phosphate, the phosphorus halides such as phosphorus trichloride, bromide, and iodide, alkyl phosphorodichloridites (RO)PCl₂, dialkylphosphorchloridites, (RO)₂PCl, dialkylphosphinochloridites, R₂PCl, alkyl alkylphosphonochloridates, (RO)(R)P(O)Cl, dialkyl phosphinochloridates, R₂P(O)Cl, and RP(O)Cl₂. Applicable corresponding sulfur derivatives include (RS)PCl₂, (RS)₂PCl, (RS)(R)P(S)Cl, and R₂P(S)Cl.

Particular phosphorus-containing compounds include ammonium phosphate, ammonium dihydrogen phosphate, diammonium hydrogen phosphate, diphenyl phosphine chloride, trimethylphosphite, phosphorus trichloride, phosphoric acid, phenyl phosphine oxychloride, trimethylphosphate, diphenyl phosphinous acid, diphenyl phosphinic acid, diethylchlorothiophosphate, methyl acid phosphate, and other alcohol-P₂O₅ reaction products.

Representative boron-containing compounds which may be used to incorporate a boron oxide modifier into the catalyst of the invention include boric acid, trimethylborate, boron oxide, boron sulfide, boron hydride, butylboron, dimethoxide, butylboric acid, dimethylboric anhydride, hexamethylborazine, phenyl boric acid, triethylborane, diborane and triphenyl boron.

Representative magnesium-containing compounds include magnesium acetate, magnesium nitrate, magnesium benzonate, magnesium propionate, magnesium 2-ethylhexoate, magnesium carbonate, magnesium formate, magnesium oxylate, magnesium bromide, magnesium hydride, magnesium lactate, magnesium laurate, magnesium oleate, magnesium palmitate, magnesium salicylate, magnesium stearate and magnesium sulfide.

Representative calcium-containing compounds include calcium acetate, calcium acetylacetonate, calcium carbonate, calcium chloride, calcium methoxide, calcium naphthenate, calcium nitrate, calcium phosphate, calcium stearate and calcium sulfate.

Representative lanthanum-containing compounds include lanthanum acetate, lanthanum acetylacetonate, lanthanum carbonate, lanthanum chloride, lanthanum hydroxide, lanthanum nitrate, lanthanum phosphate and lanthanum sulfate.

The porous crystalline material employed in the process of the invention may be combined with a variety of binder or matrix material resistant to the temperatures and other conditions employed in the process. Such materials include active and inactive materials such as clays, silica and/or metal oxides such as alumina. The latter may be either naturally occurring or in the form of gelatinous precipitates or gels including mixtures of silica and metal oxides. Use of a material which is active, tends to change the conversion and/or selectivity of the catalyst and hence is generally not preferred. Inactive materials suitably serve as diluents to control the amount of conversion in a given process so that products can be obtained economically and orderly without employing other means for controlling the rate of reaction. These materials may be incorporated into naturally occurring clays, e.g. bentonite and kaolin, to improve the crush strength of the catalyst under commercial operating conditions. Said materials, i.e. clays, oxides, etc. function as binders for the catalyst. It is desirable to prevent the catalyst from breaking down into powder-like materials. These clay and/or oxide binders have been employed normally only for the purpose of improving the crush strength of the catalyst.

Naturally occurring clays which can be composited with the porous crystalline material include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification.

In addition to the foregoing materials, the porous crystalline material can be composited with a porous matrix material such as silica-alumina, silica-magnesia, silica-zirconia, silica-thoria, silica-beryllia, silica-titania as well as ternary compositions such as silica-alumina-thoria, silica-alumina-zirconia, silica-alumia-magnesia and silica-magnesia-zirconia.

The relative proportions of porous crystalline material and inorganic oxide matrix vary widely, with the content of the former ranging from about 1 to about 90% of beads, in the range of about 2 to about 80 wt% of the composite.

Preferably, the binder material comprises silica or a kaolin clay. Procedures for preparing silica-bound zeolites, such as ZSM-5, are described in U.S. Patent Nos. 4,582,815; 5,053,374 and 5,182,242. A particular procedure for binding ZSM-5 with a silica binder involves an extrusion process.

The porous crystalline material may be combined with a binder in the form of a fluidized bed catalyst. This fluidized bed catalyst may comprise clay in the binder thereof, and may be formed by a spray-drying process to form catalyst particles having a particle size of 20-200 microns.

The catalyst of the invention may optionally be precoked. The precoking step is preferably carried out by initially utilizing the uncoked catalyst in the toluene methylation reaction, during which coke is deposited on the catalyst surface and thereafter controlled within a desired range, typically from about 1 to about 20 wt% and preferably from about 1 to 5 wt% by periodic regeneration by exposure to an oxygen-containing atmosphere at an elevated temperature.

One of the advantages of the catalyst described herein is its ease of regenerability. Thus, after the catalyst accumulates coke as it catalyzes the toluene methylation reaction, it can easily be regenerated by burning off a controlled amount of coke in a partial combustion atmosphere in a regenerator at temperatures in the range of from about 400 to about 700°C. The coke loading on the catalyst may thereby be reduced or substantially eliminated in the regenerator. If it is desired to maintain a given degree of coke loading, the regeneration step may be controlled such that the regeneration catalyst returning to the toluene methylation reaction zone is coke-loaded at the desired level.

The present process may suitably be carried out in fixed, moving or fluid catalyst beds. If it is desired to continuously control the extend of coke loading, moving or fluid bed configurations are preferred. With moving or fluid bed configurations, the extend of coke loading can be controlled by varying the severity and/or the frequency of continuous oxidative regeneration in the catalyst regenerator.

The process of the present invention is generally conducted at a temperature between 500 and 700°C, preferably between 500 and 600°C, a pressure of between 100 and 7000 kPa (1 atmosphere and 1000 psig), a weight hourly space velocity of between 0.5 and 1000, and a molar ratio of toluene to methanol (in the reactor charge) of at least 0.2, e.g., from 0.2 to 20. The process is preferably conducted in the presence of added hydrogen and/or added water such that the molar ratio of hydrogen and/or water to toluene + methanol in the feed is between 0.01 and 10.

Using the process of the invention, toluene can be alkylated with methanol so as to produce para-xylene at a selectivity of at least about 90 wt% (based on total C₈ aromatic product) at a per-pass toluene conversion of at least about 15 wt% and a trimethylbenzene production level less than 1 wt%.

The invention will now be more particularly described in the following Examples and the accompanying drawing, in which:
Figure 1 is a graph of Diffusion Parameter against para-xylene yield and para-xylene selectivity for the catalyst of Examples 10-14; and
Figures 2 and 3 are graphs of steaming temperature against n-hexane sorption capacity and Diffusion Parameter respectively for the catalysts of Example 15.

In the Examples, micropore volume (n-hexane) measurements were made on a computer controlled (Vista/Fortan) duPont 951 Thermalgravimetric analyzer. Isotherms were measured at 90°C and adsorption values taken at 75 torr n-hexane. The diffusion measurements were made on a TA Instruments 2950 Thermalgravimetric Analyzer equipped with a Therma Analysis 2000 controller, a gas switching accessory and an automatic sample changer. Diffusion measurements were made at 120°C and 8 kPa (60 torr) 2,2-dimethylbutane. Data were plotted as uptake versus square root of time. Fixed bed catalytic testing was conducted using a 1 cm (3/8") outside diameter, down-flow reactor using a two gram catalyst sample. The product distribution was analyzed with an on-line Varian 3700 GC (Supelcowax 10 capillary column, 30 m in length, 0.32 mm internal diameter, and 0.5µm film thickness.)

### Examples 1 - 5

Five samples of a composite catalyst containing 2.9 wt.% phosphorus and 10 wt% of a 450:1 SiO₂/Al₂O₃ ZSM-5 in a binder comprising silica-alumina and clay were steamed for 0.75 hours, one atmosphere steam at 975°C (Example 1), 1000°C (Example 2), 1025 °C (Example 3), 1050°C (Example 4) and 1075°C (Example 5). The effect of steaming temperature on the n-hexane sorption capacity (Q) compared to that of the unsteamed catalyst (10.7 mg/g) and on the Diffusion Parameter (D/r²x 10⁶) is summarized in Table 1 below.

Samples of the five steamed catalysts were then used in toluene methylation tests on a feed comprising toluene, methanol and water such that toluene/MeOH molar ration = 2 and H₂O/HC molar ration = 2 (where HC = toluene + methanol). The tests were conducted at 600°C, 380 kPa, (40 psig) and HC WHSV = 4 in the presence of hydrogen such that H₂/HC molar ratio = 2. The results of Examples 2 - 5 are summarized in Table 2.

**Table 1**

| Catalyst ID | Steaming Temp. (°C) | Q(n-C₆, mg/g) | % retention of initial sorption capacity | D/r² sec⁻¹(x10⁶) |
|---|---|---|---|---|
| Ex. 1 | 975 | 10.3 | 96 | 21.2 |
| Ex. 2 | 1000 | 9.7 | 91 | 16.4 |
| Ex. 3 | 1025 | 9.1 | 85 | 10.2 |
| Ex. 4 | 1050 | 8.4 | 79 | 3.2 |
| Ex. 5 | 1075 | 6.5 | 61 | 0.3 |

**Table 2**

| | Example 2 | Example 3 | Example 4 | Example 5 |
|---|---|---|---|---|
| Temp, °C | 600.0 | 600.0 | 600.0 | 600.0 |
| Pressure, (psig) kPa | (40.5) 279 | (42.8) 295 | (40.3) 278 | (43.2) 298 |
| WHSV | 4.0 | 4.0 | 4.0 | 4.0 |
| Time on Stream, hr | 4.8 | 20.6 | 5.1 | 5.0 |
| | | | | |

| Product Distribution (wt %) | | | | |
|---|---|---|---|---|
| C5- | 1.42 | 0.73 | 0.99 | 1.44 |
| MeOH | 0.02 | 0.17 | 0.21 | 1.85 |
| BENZENE | 0.25 | 0.13 | 0.20 | 0.27 |
| TOLUENE | 61.82 | 62.41 | 66.27 | 81.63 |
| EB | 0.06 | 0.06 | 0.06 | 0.04 |
| P-XYL | 30.38 | 32.21 | 30.52 | 14.26 |
| M-XYL | 3.17 | 2.00 | 0.68 | 0.13 |
| O-XYL | 1.37 | 0.94 | 0.33 | 0.11 |
| ETOL | 0.30 | 0.33 | 0.31 | 0.14 |
| TMBENZENE | 1.05 | 0.92 | 0.37 | 0.08 |
| C10+ | 0.15 | 0.10 | 0.04 | 0.04 |
| | 100.00 | 100.00 | 100.00 | 100.00 |
| | | | | |

| Performance Data | | | | |
|---|---|---|---|---|
| Toluene Conv. % | 33.21 | 32.56 | 28.39 | 11.80 |
| MeOH Conv. % | 99.71 | 97.76 | 97.76 | 75.12 |
| MeOH Utilization, mol% | 62.08 | 63.72 | 57.53 | 34.26 |
| p-Xylene Selectivity, % | 86.99 | 91.64 | 96.80 | 98.35 |
| Xylene Yield on Toluence, wt% | 37.73 | 37.98 | 34.07 | 15.67 |
| P-Xylene Yield on Tol, wt% | 32.8 | 34.8 | 33.0 | 15.4 |
| Xylenes/Aromatic Product, wt% | 95.1 | 95.8 | 97.0 | 96.2 |

From Table 2 it will be seen, with the catalyst of Examples 1 - 5, steaming at a temperature in excess of 1000 °C was necessary to reduce the D/r² value below 15 and with the Example 2 catalyst (steamed at 1000 °C to a D/r² value of 16.4), the p-selectivity of the catalyst was below 87%. As the steaming temperature increased above 1000 °C to 1075 °C, the para-xylene selectivity increased but with the catalyst steamed at 1075 °C this was accompanied by a significant decrease in the para-xylene yield and the methanol utilization (moles of xylene produced/moles of methanol converted).

### Examples 6 - 9

A second composite catalyst containing 4.5 wt% phosphorus and 10 wt% of a 450:1 SiO₂/AL₂O₃ ZSM-5 in a binder comprising silica-alumina and clay was divided into four samples which were steamed for 0.75 hours, one atmosphere steam at 950°C (Example 6), 975°C (Example 7), 1000°C (Example 8), and 1025°C (Example 9). The effect of steaming temperature on the n-hexane sorption capacity (Q) and Diffusion Parameter (D/r²x10⁶) of these catalysts is summarized in Table 3 below.

Samples of the four steamed catalysts were then used in toluene methylation tests conducted as in Examples 2 - 5 with the HC WHSV = 4. The results of Examples 6 - 9 are summarized in Table 4.

**Table 3**

| Catalyst ID | Steaming Temp. (°C) | Q(n-C₆, mg/g) | % retention of initial sorption capacity | D/r² sec⁻¹(x10⁶) |
|---|---|---|---|---|
| Unsteamed Sample | | 12.7 | | 21.7 |
| Ex. 6 | 950 | 9.4 | 74 | 6.3 |
| Ex. 7 | 975 | 7.2 | 57 | 5.3 |
| Ex. 8 | 1000 | 7.9 | 62 | 1.92 |
| Ex. 9 | 1025 | 7.0 | 55 | 0.84 |

**Table 4**

| | Example 6 | Example 7 | Example 8 | Example 9 |
|---|---|---|---|---|
| Temp, °C | 600.0 | 600.0 | 600 | 600 |
| Pressure, (psig) kPa | (44.3) 305 | (43.1) 297 | (45.3) 312 | (42.0) 290 |
| WHSV | 4.0 | 4.0 | 4.0 | 4.0 |
| Time on Stream, hr | 6.3 | 5.9 | 5.9 | 5.9 |
| | | | | |

| **Product Distribution (wt %)** | | | | |
|---|---|---|---|---|
| C5- | 1.70 | 1.68 | 1.69 | 1.57 |
| MeOH | 1.16 | 0.35 | 1.15 | 2.43 |
| BENZENE | 0.24 | 0.20 | 0.24 | 0.22 |
| TOLUENE | 65.17 | 67.30 | 73.76 | 83.83 |
| EB | 0.06 | 0.05 | 0.05 | 0.03 |
| P-XYL | 29.99 | 28.55 | 22.09 | 11.50 |
| M-XYL | 1.16 | 0.72 | 0.34 | 0.13 |
| O-XYL | 0.52 | 0.35 | 0.21 | 0.11 |
| ETOL | 0.31 | 0.28 | 0.21 | 0.11 |
| TMBBENZENE | 0.63 | 0.46 | 0.27 | 0.07 |
| C10+ | 0.06 | 0.05 | 0.00 | 0.00 |
| | 100.00 | 100.00 | 100.00 | 98.43 |
| | | | | |

| **Performance Data** | | | | |
|---|---|---|---|---|
| Toluene Conv. % | 29.73 | 27.44 | 20.47 | 9.62 |
| MeOH Conv. % | 97.81 | 95.17 | 84.14 | 66.42 |
| MeOH Utilization, mol% | 59.02 | 59.02 | 49.02 | 32.20 |
| p-Xylene Selectivity, % | 94.70 | 96.38 | 97.58 | 97.95 |
| Xylene Yield on Tol, wt% | 34.14 | 31.93 | 24.40 | 12.66 |
| P-Xylene Yield on Tol, wt% | 32.3 | 30.8 | 23.8 | 12.4 |
| Xylenes/Aromatic Product, | 96.1 | 96.6 | 96.7 | 96.5 |
| wt% | | | | |

### Comparative Example A

ZSM-5 crystals were prepared according to the method set forth in Example 33 of the Butter et al U.S. Patent No. 3,965,209. The ZSM-5 had a silica to alumina molar ratio of about 70 to 1 and was combined with an alumina binder in a ratio of 65 weight % zeolite and 35 weight % binder.

The bound, phosphorus-free catalyst had an n-hexane sorption capacity, Q of 74.4 mg/g and a Diffusion Parameter for 2,2-dimethylbutane of 740 sec⁻¹. The catalyst was steamed at 950°C for 65 hours at atmospheric pressure (100 kPa) in 100% steam which reduced its n-hexane sorption capacity, Q to 32.4 mg/g, or 44% of the initial capacity, and its Diffusion Parameter for 2,2-dimethylbutane to 1.72 sec⁻¹. The steamed catalyst was then subjected to catalytic testing in the same manner as Examples 1-9. In particular, experiments were conducted at 600°C, 40 psig (380 kPa), H2/HC=2, H2O/HC=2, WHSV=4 with a toluene/MeOH=2 feed. The results are summarized in Table 5, which provides data for an average analysis of three samples taken at 28.53, 33.32 and 37.22 hours on stream.

**Table 5**

| Temp, °C | 600 |
|---|---|
| Pressure, (psig) kPa | (39.63) 273 |
| WHSV | 4.00 |
| Time on Stream, hr | 33.0 |
| | |

| Product Distribution (wt %) | |
|---|---|
| C5- | 0.36 |
| DME | 0.09 |
| MeOH | 1.35 |
| BENZENE | 0.19 |
| TOLUENE | 71.91 |
| EB | 0.05 |
| P-XYL | 21.40 |
| M-XYL | 1.91 |
| O-XYL | 1.18 |
| ETOL | 0.17 |
| TMBENZENE | 1.29 |
| C10+ | 0.09 |
| | 100.00 |
| | |

| Performance Data | |
|---|---|
| Toluene Conv. % | 22.30 |
| MeOH Conv. % | 80.74 |
| MeOH Utilization, mol% | 53.79 |
| p-Xylene Selectivity, % | 87.37 |
| Xylene Yield on Toluene, wt% | 26.47 |
| P-Xylene Yield on Toluene, wt% | 23.1 |
| Xylenes/Aromatic Product, wt % | 93.2 |

The data in Table 5 show that in Comparative Example A, although the toluene conversion was 22.30%, the para-selectivity was only 87.37%, the methanol conversion was only 80.74% and the wt% xylenes based on the total aromatic product was only 93.2. Furthermore, the yield of the unwanted by-product, trimethylbenzene, was 1.29 wt%.

### Examples 10 - 14

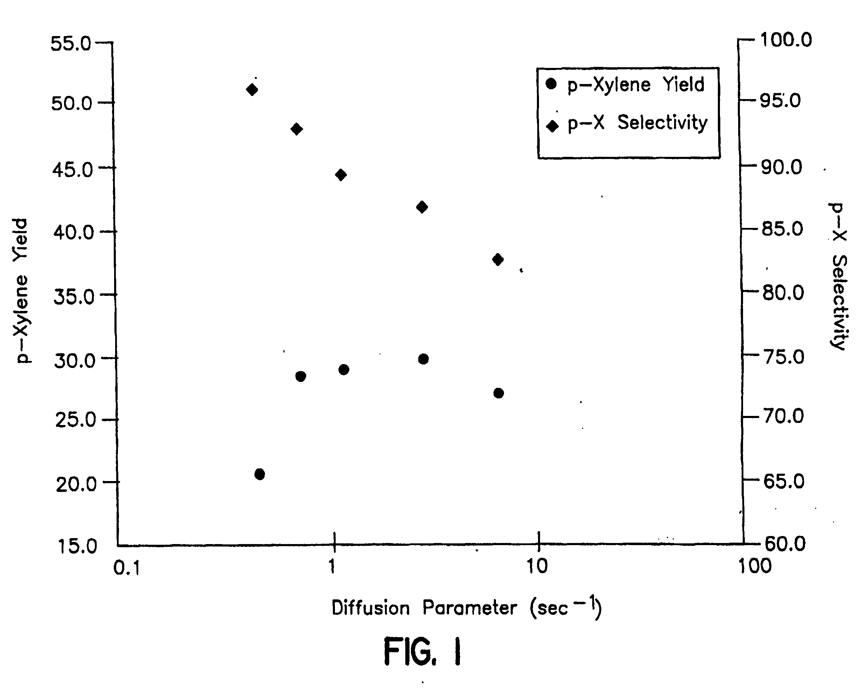

A series of fluid bed catalysts were produced containing about 4 wt% phosphorus and 25 wt% of a 450:1 SiO₂/Al₂O₃ ZSM-5 in a binder comprising kaolin clay. The catalysts were steamed for 0.75 hours at varying temperatures between 1025 and 1060°C and were used to effect the alkylation of toluene with methanol in a bench-scale fluid bed reactor in the absence of cofed hydrogen. Details of the test and the results obtained are summarized in Table 6 and Figure 1. It will be seen that, as the Diffusion Parameter of the catalyst decreased with increasing steaming severity, the para-xylene selectivity increased generally linearly whereas the para-xylene yield increased to a maximum at a D/r² value of 1-2 x 10⁻⁶ sec⁻¹ before decreasing again.

**Table 6**

| Example No. | 10 | 11 | 12 | 13 | 14 |
|---|---|---|---|---|---|
| **Catalyst Properties** | | | | | |
| Phosphorus, wt% | 3.9 | 3.8 | 4.1 | 4.1 | 3.8 |
| Steaming Temp. C | 1025 | 1031 | 1033 | 1033 | 1060 |
| D/r², sec⁻¹ (x10⁶) | 1.14 | 0.71 | 2.81 | 6.5 | 0.45 |
| Q, (n-C6) mg/g | 19.7 | 19 | 14.1 | 14.2 | 17.8 |
| Parent Q, mg/g | 21.6 | 21.6 | 17.1 | 20.7 | 21.9 |
| | | | | | |

| **Reaction Conditions** | | | | | |
|---|---|---|---|---|---|
| Feed Toluene/Methanol (mol/mol) | 2.08 | 2.03 | 19.3 | 2.06 | 2.17 |
| Feed H2O/HC (mol/mol) | 0.47 | 0.46 | 0.63 | 0.63 | 0.51 |
| Reactor Temp, (F) °C | (1105) 596 | (1107) 597 | (1113) 601 | (1108) 598 | (1110) 599 |
| Reactor P, (psig) kPa | (20.6) 142 | (20.7) 143 | (20.8) 143 | (21.7) 150 | (20.7) 143 |
| HC WHSV | 1.71 | 1.75 | 1.75 | 1.72. | 1.71 |
| Time on Stream, Hrs | 10 | 10 | 10 | 10 | 2 |
| | | | | | |

| **Feed Composition, wt%** | | | | | |
|---|---|---|---|---|---|
| MeOH | 12.81 | 13.12 | 13.20 | 12.50 | 12.26 |
| Toluene | 76.76 | 76.53 | 73.19 | 73.93 | 73.51 |
| H20 | 10.43 | 10.35 | 13.61 | 13.57 | 11.23 |
| | | | | | |

| **Product Composition, wt%** | | | | | |
|---|---|---|---|---|---|
| C5- | 1.46 | 16.5 | 1.67 | 1.52 | 2.41 |
| MeOH | 0.01 | 0.03 | 0.09 | 0.01 | 0.29 |
| Benzene | 0.40 | 0.31 | 0.33 | 0.34 | 0.32 |
| Toluene | 54.60 | 55.89 | 50.81 | 52.41 | 62.16 |
| EB | 0.05 | 0.05 | 0.05 | 0.05 | 0.04 |
| p-Xylene | 22.15 | 21.68 | 21.72 | 19.85 | 15.70 |
| m-Xylene | 1.87 | 1.16 | 2.33 | 2.94 | 0.42 |
| o-Xytene | 0.78 | 0.50 | 1.00 | 1.25 | 0.02 |
| Styrene | 0.02 | 0.02 | 0.02 | 0.02 | 0.02 |
| E-Toluene | 0.25 | 0.25 | 0.23 | 0.23 | 0.20 |
| TMBezene | 0.52 | 0.48 | 0.70 | 0.72 | 0.24 |
| C10+ | 0.28 | 0.34 | 0.31 | 0.30 0.39 | |
| H20 | 17.61 | 17.64 | 20.74 | 20.36 | 17.61 |
| | | | | | |

| **Performance Data** | | | | | |
|---|---|---|---|---|---|
| Toluene Conv, % | 28.9 | 27.0 | 30.6 | 29.1 | 18.8 |
| MeOH Conv, % | 99.9 | 99.8 | 99.3 | 99.9 | 97.6 |
| MeOH Utilization, mol% | 58.5 | 53.8 | 57.7 | 58.1 | 41.1 |
| p-Xylene Selectivity, % | 89.3 | 92.9 | 86.7 | 82.6 | 96.2 |
| Xylene Yield on Tol, wt% | 32.3 | 30.5 | 34.2 | 32.5 | 21.3 |
| p-Xylene Yield on Tol, wt% | 28.9 | 28.3 | 29.7 | 26.8 | 20.5 |
| Xylenes/Aromatic Products, wt% | 95.7 | 95.3 | 95.0 | 94.8 | 94.8 |

### Example 15

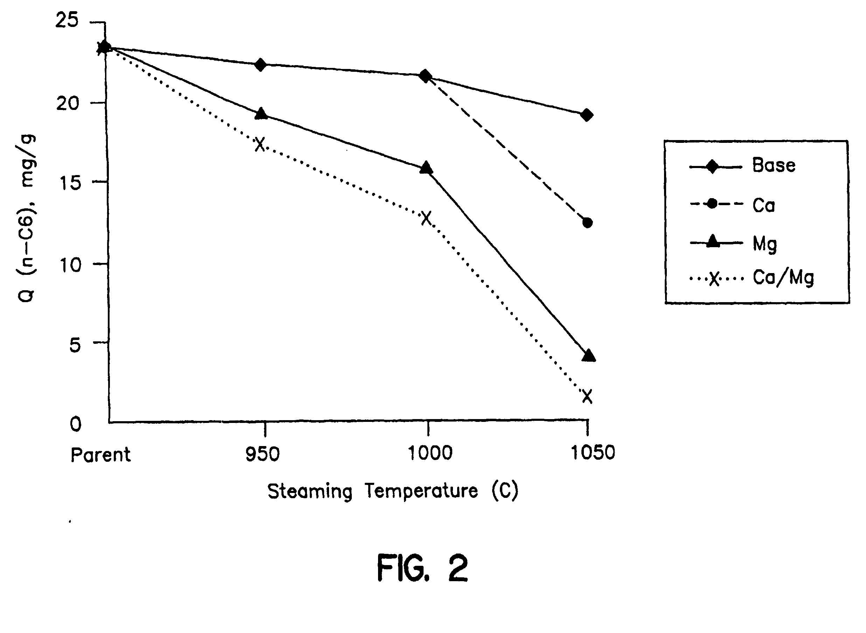

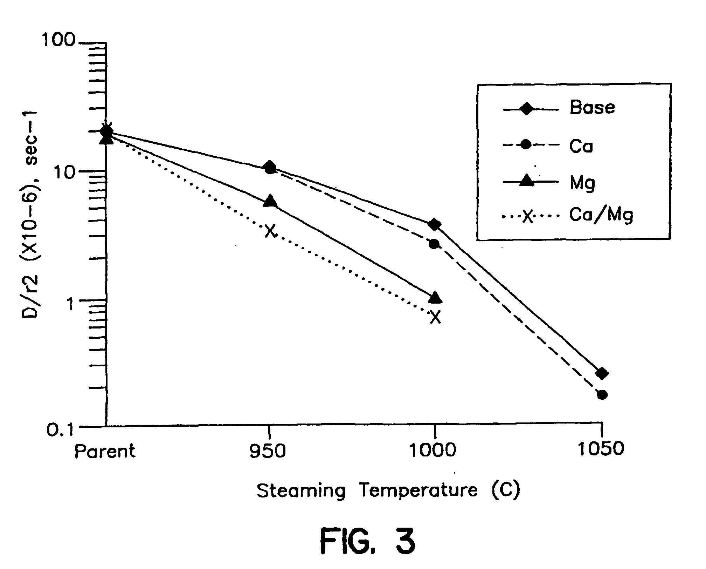

A series of three catalysts similar to those of Examples 10 - 14 (25wt% ZSM-5 of 450:1 silica/alumina ratio, 75 wt% clay binder with additional 4 wt% phosphorus) was prepared by doping respectively with calcium (2000 ppmw added), magnesium (5000ppmw added), and both calcium and magnesium (2000 ppmw CA/5000 ppmw MG added). Slurries were prepared by mixing together components in the following order: ZSM-5 slurry, phosphoric acid, calcium/magnesium (from nitrate salts), and clay. Catalysts were spray dried and the air calcined at 540°C for 3 hours. Three samples of each catalyst were then steamed for 45 minutes in 1 atmosphere steam at 950°C, 1000°C, and 1050°C, respectively. The n-hexane sorption capacity and the Diffusion Parameter of the catalysts are plotted against steming temperature in Figures 2 and 3. The presence of magnesium (and calcium to a lesser extent) decreases the steaming temperature required to produce a catalyst with a given Diffusion Parameter. These data show that combinations of oxide modifiers can effectively be used to produce th desired catalyst.

### Example 16

A comparison was made between a catalyst similar to those used in Examples 10 - 14, in which the initial zeolite had a silica/alumina molar ratio of 450, and a catalyst produced from ZSM-5 having an initial silica/alumina molar ratio of 26. In each case the catalyst contained about 4 wt% phosphorus and 25 wt% of ZSM-5 in a binder comprising kaolin clay and was steamed for 45 minutes at >1000°C before being used to effect the alkylation of toluene with methanol in a fixed bed microunit. The results are summarized in Table 7 from which it will be seen that the 26:1 material had significantly lower activity (as indicated by lower WHSV necessary to achieve comparable toluene conversion), lower para-selectivity and lower methanol utilization than the 450:1 material.

**Table 7**

| **Catalyst Properties** | | | |
|---|---|---|---|
| Percent ZSM-5 | 25 | | 25 |
| Si:Al Ratio | 450 | | 26 |
| Steaming Temperature, °C | 1051 | | 1016 |
| | | | |

| **Reaction Conditions** | | | |
|---|---|---|---|
| Temperature, °C | 600 | | 585 |
| Pressure, (psig) kPa | (40) 276 | | (40) 276 |
| WHSV | 8.0 | | 2.50 |
| Tol/MeOH (mol/mol) | 2.0 | | 2.00 |
| H2/HC (mol/mol) | 2.0 | | 2.00 |
| H2O/HC (mol/mol) | 2.0 | | 2.00 |
| Time on Stream, hr | 14.30 | | 6.00 |
| | | | |

| **Products Composition, wt%** | | | |
|---|---|---|---|
| C5- | 0.05 | | 2.12 |
| DME | 0.00 | | 0.00 |
| MeOH | 0.40 | | 0.32 |
| BENZENE | 0.13 | | 0.23 |
| TOLUENE | 64.42 | | 65.83 |
| EB | 0.06 | | 0.05 |
| P-XYLENE | 32.66 | | 27.82 |
| M-XYLENE | 0.94 | | 1.81 |
| O-XYLENE | 0.42 | | 0.75 |
| ETHYL TOLUENE | 0.33 | | 0.25 |
| TMBENZENE | 0.56 | | 0.76 |
| C10+ | 0.04 | | 0.06 |
| | | | |

| **Performance Data** | | | |
|---|---|---|---|
| Toluene Conv., % | 30.68 | | 29.16 |
| MeOH Conv., % | 94.30 | | 95.43 |
| MeOH Utilization, mol% | 67.37 | | 59.46 |
| p-Xylene Selctivity, % | 96.02 | | 91.58 |
| Xylene Yield on Tol, wt% | 36.61 | | 32.69 |
| p-Xylene Yield on Tol, % | 35.2 | | 29.9 |
| Xylenes/Aromatic Products, wt% | 96.8 | | 95.7 |

### Examples 17 and 18

Two composite catalysts were produced containing 10wt% of 450:1 SiO₂/Al₂O₃ ZSM-5 in kaolin clay matrix which in one case also contained 2.8 wt% phosphorus (Example 17) and the other case did not contain phosphorus (Example 18). Each catalyst was steamed at 1010°C for 0.75 hour and was then used to effect the alkylation of toluene with methanol in a bench-scale fluid bed reactor containing 80 grams of catalyst. The properties of the steamed catalysts and the results of the toluene alkylation tests are shown in Table 8 below.

From Table 8 it will be seen that the Diffusion Parameter, D/r² of the phosphorus-free catalyst of Example 18 remained high after steaming. In addition, it will be seen that the para-xylene selectivity and yield of the phosphorus containing catalyst of Example 17 were significantly higher that those of the phosphorus-free catalyst of Example 18.

**Table 8**

| Example | 17 | 18 |
|---|---|---|
| **Catalyst Properties** | | |
| Phosphorus, wt% | 2.8 | 0 |
| D/r², sec⁻¹ (x10⁸) | 2.54 | 36.28 |
| Q, (n-C₆) mg/g | 8.1 | 8.8 |
| Parent Q, mg/g | 8.4 | 10.6 |
| | | |

| **Feed Composition, wt%** | | |
|---|---|---|
| MeOH | 12.82 | 13.15 |
| Toluene | 75.61 | 75.61 |
| H20 | 11.57 | 11.24 |
| TOTAL | 100 | 100 |
| | | |

| **Reaction Conditions** | | |
|---|---|---|
| Feed Toluene/Methanol (mol/mol) | 2.05 | 2.00 |
| Feed H2O/HC (mol/mol) | 0.52 | 0.51 |
| Reactor Temp, (F) °C | (1107) 597 | (1108) 598 |
| Reactor Pressure, (psig) kPa | (19) 131 | (21.6) 149 |
| HC WHSV | 1.72 | 1.74 |
| Time on Stream, Hrs | 6 | 6 |
| | | |

| **Product Composition, wt%** | | |
|---|---|---|
| C5- | 1.66 | 1.53 |
| MeOH | 0.34 | 0.33 |
| Benzene | 0.26 | 0.31 |
| Toluene | 55.09 | 54.33 |
| EB | 0.04 | 0.03 |
| p-Xylene | 20.08 | 15.94 |
| m-Xylene | 1.57 | 4.83 |
| o-Xylene | 0.72 | 2.3 |
| Styrene | 0.02 | 0.01 |
| E-Toluene | 0.23 | 0.17 |
| TMBezene | 0.61 | 1.49 |
| C10+ | 0.29 | 0.25 |
| H20 | 19.09 | 18.48 |
| | 100 | 100 |
| | | |

| **Performance Data** | | |
|---|---|---|
| Toluene Conv., % | 27.1 | 28.1 |
| MeOH Conv., % | 97.3 | 97.5 |
| MeOH Utilization, mol% | 54.1 | 54.3 |
| p-Xylene Selctivity, % | 89.8 | 69.1 |
| Xylene Yield on Tol, wt% | 29.6 | 30.5 |
| p-Xylene Yield on Tol, % | 26.6 | 21.1 |
| Xylenes/Aromatic Products, wt% | 94.9 | 92.2 |

### Examples 19 - 21

A base catalyst particle was prepared by spray-drying a mixture of ZSM-5 having a silica-alumina molar ratio of 450:1, kaolin clay and silica. After rotary calcination at 650°C (1200°F), the final composition of the catalyst was 40 wt% ZSM-5, 30 wt% kaolin and 30 wt% silica. The calcined catalyst was divided into three samples, which were impregnated by the incipient wetness techniques with solutions containing boron (Example 19), magnesium (Example 20) and lanthanum (Example 21) respectively and giving the following compositions:
a) boron-containing solution
   - 20 gm boric acid
      800 gm distilled water
      8gm 30 wt% ammonium hydroxide
b) magnesium-containing solution
   - 20gm magnesium nitrate hexahydrate
      240 gm distilled water
c) lanthanum-containing solution
   - 20gm lanthanum nitrate hexahydrate
      80gm distilled water.

In each case, impregnation was conducted by incipient wetness by adding 0.79 gm of the appropriate solution to a catalyst sample, after which the sample was dried at 150°C for 2 hours and then air calcined at 550°C for 4 hours to convert the ammonium and nitrate salts to oxides. The oxide-modified catalysts were then heated in 1 atmosphere steam at 1000°C. Table 9 lists the oxide loading on each catalyst on an elemental basis and the n-hexane adsorption capacity (Q in mg/g) and Diffusion Parameter (D/r²x10⁶ sec⁻¹) of the unsteamed and steamed catalysts.

**Table 9**

| Unsteamed Catalyst | Oxide Loading | Q (n-C₆, mg/g) | D/r² sec⁻¹ (x10⁶) |
|---|---|---|---|
| Example 19 | 0.2 wt% boron | 52.6 | 17 |
| Example 20 | 0.5 wt% magnesium | 42.7 | 24.2 |
| Example 21 | 4.9 wt% lanthanum | 39.8 | 19.9 |
| | | | |

| Steamed Catalyst | Oxide Loading | Q (n-C₆, mg/g) | D/r² sec⁻¹ (x10⁶) |
|---|---|---|---|
| Example 19 | 0.2 wt% boron | 32.7 | 2.6 |
| Example 20 | 0.5 wt% magnesium | 37.3 | 9.4 |
| Example 21 | 4.9 wt% lanthanum | 31.1 | 1.4 |

The steamed catalysts of Examples 19 and 21 were then tested in the alkylation of toluene with methanol under the conditions and with the results listed in Table 10.

**Table 10**

| Example | 19 | 21 |
|---|---|---|
| **Reaction Conditions** | | |
| Temperature, °C | 592 | 592 |
| Pressure, (psia) kPa | (16) 110 | (16) 110 |
| WHSV | 3 | 4 |
| Time on stream, minutes | 402 | 6 |

| **Product Composition, wt%** | | |
|---|---|---|
| C5- | 2.33 | 2,47 |
| Methanol | 0.59 | 3.18 |
| Benzene | 0.07 | 0.04 |
| Toluene | 64.6 | 67.54 |
| Ethylbenzene | 0.06 | 0.06 |
| P-xylene | 28.05 | 23.02 |
| M-xylene | 1.81 | 1.47 |
| O-xylene | 0.75 | 0.61 |
| Ethyltoluene | 0.31 | 0.30 |
| Trimethylbenzene | 1.08 | 0.78 |
| C10+ | 0.34 | 0.52 |

| **Performance Data** | | |
|---|---|---|
| % of total xylenes | | |
| Para | 91.6 | 91.71 |
| Meta | 5.90 | 5.85 |
| Ortho | 2.46 | 2.44 |
| Total xylenes, wt% | 30.61 | 25.10 |
| Xylenes/total aromatic product | 94.46 | 93.81 |
| Toluene conversion | 30.2 | 25.9 |
| Methanol conversion | 96.3 | 79.9 |
| Methanol utilization | 60 | 60 |

## Claims

1. A process for the selective production of para-xylene which comprises reacting toluene with methanol under alkylation conditions in the presence of a catalyst comprising a porous crystalline material having a Diffusion Parameter for 2,2 dimethylbutane of 0.1 - 15 sec⁻¹ when measured at a temperature of 120 °C, and a 2,2 dimethylbutane pressure of 8 kPa (60 torr), and wherein said porous crystalline material having a Diffusion Parameter of greater than 15 sec⁻¹ has undergone prior treatment with steam at a temperature in excess of 1000 °C to adjust the Diffusion Parameter of said material to 0.1 - 15 sec⁻¹ and the steaming reduces the pore volume of the catalyst when measured by n-hexane adsorption at a temperature of 90 °C and a n-hexane pressure of 10 kPa (75 torr) to not less than 50% of that of the catalyst prior to said steam treatment.

2. The process of claim 1, wherein said diffusion parameter of said porous crystalline material is 0.5-10 sec⁻¹.

3. The process of claim 1, wherein said porous crystalline material has undergone prior treatment with steam for between 10 minutes and 100 hours.

4. The process of claim 1, wherein the catalyst contains at least one oxide modifier selected from oxides of elements of Groups IIA, IIIA, IIIB, IVA, IVB, VA and VIA of the Periodic Table.

5. The process of claim 1, wherein the catalyst contains at least one oxide modifier selected from oxides of boron, magnesium, calcium, lanthanum and phosphorus.

6. The process of claim 1, wherein the catalyst contains 0.05 to 20 wt% of the oxide modifier based on the elemental modifier.

7. The process of claim 1, wherein the catalyst contains 0.1 to 10 wt% of the oxide modifier based on the elemental modifier.

8. The process of claim 1, wherein the catalyst has an average particle size of 20 to 200 microns.

9. The process of claim 1, wherein the porous crystalline material is an aluminosilicate zeolite.

10. The process of claim 9, wherein said zeolite is ZSM-5 or ZSM-11.

11. The process of claim 1, wherein said alkylation conditions include a temperature between 500 and 700°C, a pressure of between 100 and 7000 kPa (1 atmosphere to 1000 psig), a weight hourly space velocity between 0.5 and 1000 and a molar ratio of toluene to methanol of at least 0.2.

12. The process of claim 1, wherein said alkylation is conducted in the presence of added hydrogen and/or water such that the molar ratio of hydrogen and/or water to toluene + methanol in the feed is 0.01 to 10.

13. A method for producing a catalyst for use in the selective production of para-xylene by reacting toluene with methanol, said method comprising the steps of:
(a) starting with a porous crystalline material giving a Diffusion Parameter for 2,2 dimethylbutane in excess of 15 sec⁻¹ when measured at a temperature of 120 °C, and a 2,2 dimethylbutane pressure of 8 kPa (60 torr);
and
(b) contacting the material of step (a) with steam at a temperature of at least 1000 °C to reduce the Diffusion Parameter thereof for 2,2 dimethylbutane to about 0.1 - 15 sec⁻¹ when measured at a temperature of 120 °C, and a 2,2 dimethylbutane pressure of 8 kPa (60 torr), the micropore volume of the steamed material when measured by n-hexane adsorption at a temperature of 90 °C and a n-hexane pressure of 10 kPa (75 torr) being at least 50% of the catalyst prior to said steam treatment.

14. The method of claim 13, wherein the porous crystalline material is combined with a source of at least one oxide modifier selected from oxides of elements of Groups IIA, IIIA, IIIB, IVA, IVB, VA and VIA of the Periodic Table prior to step (b).

15. The method of claim 13, wherein the porous crystalline material is combined with a source of at least one oxide modifier selected from oxides of boron, magnesium, calcium, lanthanum and phosphorus prior to step (b).

16. The method of claim 13, wherein the porous crystalline material used in step (a) is an aluminosilicate zeolite having a constraint index of 1 to 12.

17. The method of claim 16, wherein the zeolite has a silica to alumina molar ratio of at least 250.

18. The method of claim 13, wherein step (b) is conducted at a temperature of at least 1000°C for 10 minutes to 100 hours.

## Patentansprüche

1. Verfahren zur selektiven Erzeugung von p-Xylol, das die Umsetzung von Toluol mit Methanol unter Alkylierungsbedingungen in Gegenwart eines Katalysators umfaßt, der ein poröses kristallines Material mit einem Diffusionsparameter für 2,2-Dimethylbutan von 0,1 bis 15 s⁻¹ aufweist, wenn bei einer Temperatur von 120°C und einem Druck von 2,2-Dimethylbutan von 8 kPa (60 Torr) gemessen wird, und wobei das poröse kristalline Material mit einem Diffusionsparameter von mehr als 15 s⁻¹ einer Vorbehandlung mit Dampf bei einer Temperatur von mehr als 1000°C unterzogen worden ist, um den Diffusionsparameter des Materials bei 0,1 bis 15 s⁻¹ einzustellen und die Dampfbehandlung das Porenvolumen des Katalysators, wenn durch Adsorption von n-Hexan bei einer Temperatur von 90°C und einem Druck von n-Hexan von 10 kPa (75 Torr) gemessen wird, auf nicht weniger als 50 % von dem des Katalysators vor dieser Dampfbehandlung verringert.

2. Verfahren nach Anspruch 1, wobei der Diffusionsparameter des porösen kristallinen Materials 0,5 bis 10 s⁻¹ beträgt.

3. Verfahren nach Anspruch 1, wobei das poröse kristalline Material 10 Minuten bis 100 Stunden einer Vorbehandlung mit Dampf unterzogen worden ist.

4. Verfahren nach Anspruch 1, wobei der Katalysator mindestens ein Modifikationsmittel aus einem Oxid enthält, das aus Oxiden der Elemente der Gruppen IIA, IIIA, IIIB, IVA, IVB, VA und VIA des Periodensystems ausgewählt ist.

5. Verfahren nach Anspruch 1, wobei der Katalysator mindestens ein Modifikationsmittel aus einem Oxid enthält, das aus Oxiden von Bor, Magnesium, Calcium, Lanthan und Phosphor ausgewählt ist.

6. Verfahren nach Anspruch 1, wobei der Katalysator, bezogen auf das elementare Modifikationsmittel, 0,05 bis 20 Gew.-% des Modifikationsmittels aus einem Oxid enthält.

7. Verfahren nach Anspruch 1, wobei der Katalysator, bezogen auf das elementare Modifikationsmittel, 0,1 bis 10 Gew.-% des Modifikationsmittels aus einem Oxid enthält.

8. Verfahren nach Anspruch 1, wobei der Katalysator eine mittlere Partikelgröße von 20 bis 200 µm hat.

9. Verfahren nach Anspruch 1, wobei das poröse kristalline Material ein Aluminosilicatzeolith ist.

10. Verfahren nach Anspruch 9, wobei der Zeolith ZSM-5 oder ZSM-11 ist.

11. Verfahren nach Anspruch 1, wobei die Alkylierungsbedingungen eine Temperatur von 500 bis 700°C, einen Druck von 100 bis 7000 kPa (1 Atmosphäre bis 1000 psig), eine stündliche Gewichts-Raum-Geschwindigkeit von 0,5 bis 1000 und ein Molverhältnis zwischen Toluol und Methanol von mindestens 0,2 einschließen.

12. Verfahren nach Anspruch 1, wobei die Alkylierung in Gegenwart von zugesetztem Wasserstoff und/oder Wasser durchgeführt wird, so daß das Molverhältnis zwischen Wasserstoff und/oder Wasser und Toluol + Methanol in der Beschickung 0,01 bis 10 beträgt.

13. Verfahren zur Herstellung eines Katalysators für die Verwendung bei der selektiven Herstellung von p-Xylol durch die Reaktion von Toluol mit Methanol, wobei das Verfahren die Schritte umfaßt:
(a) es wird von einem porösen kristallinen Material ausgegangen, das einen Diffusionsparameter für 2,2-Dimethylbutan von mehr als 15 s⁻¹ ergibt, wenn bei einer Temperatur von 120°C und einem Druck von 2,2-Dimethylbutan von 8 kPa (60 Torr) gemessen wird; und
(b) das Material vom Schritt (a) wird mit Dampf mit einer Temperatur von mindestens 1000°C in Kontakt gebracht, um dessen Diffusionsparameter für 2,2-Dimethylbutan auf etwa 0,1 bis 15 s⁻¹ zu verringern, wenn bei einer Temperatur von 120°C und einem Druck von 2,2-Dimethylbutan von 8 kPa (60 Torr) gemessen wird, wobei das Mirkoporenvolumen des der Dampfbehandlung unterzogenen Materials, wenn durch die Adsorption von n-Hexan bei einer Temperatur von 90°C und einem Druck von n-Hexan von 10 kPa (75 Torr) gemessen wird, mindestens 50 % von dem des Katalysators vor dieser Dampfbehandlung beträgt.

14. Verfahren nach Anspruch 13, wobei das poröse kristalline Material vor dem Schritt (b) mit einer Quelle von zumindest einem Modifikationsmittel aus einem Oxid kombiniert wird, das aus Oxiden der Elemente der Gruppen IIA, IIIA, IIIB, IVA, IVB, VA und VIA des Periodensystems ausgewählt ist.

15. Verfahren nach Anspruch 13, wobei das poröse kristalline Material vor dem Schritt (b) mit einer Quelle von zumindest einem Modifikationsmittel aus einem Oxid gemischt wird, das aus Oxiden von Bor, Magnesium, Calcium, Lanthan und Phosphor ausgewählt ist.

16. Verfahren nach Anspruch 13, wobei das poröse kristalline Material, das im Schritt (a) verwendet wird, ein Aluminosilicatzeolith mit einem Zwangsindex von 1 bis 12 ist.

17. Verfahren nach Anspruch 16, wobei der Zeolith ein Molverhältnis zwischen Siliciumdioxid und Aluminiumoxid von mindestens 250 aufweist.

18. Verfahren nach Anspruch 13, wobei der Schritt (b) 10 Minuten bis 100 Stunden bei einer Temperatur von mindestens 1000°C durchgeführt wird.

## Revendications

1. Procédé pour la production sélective de para-xylène qui consiste à faire réagir le toluène avec le méthanol dans des conditions d'alkylation en présence d'un catalyseur comprenant un matériau cristallin poreux ayant un paramètre de diffusion pour le 2,2-diméthylbutane de 0,1 à 15 s⁻¹ lorsqu'il est mesuré à une température de 120°C et une pression de 2,2-diméthylbutane de 8 kPa (60 torr) et dans lequel ledit matériau cristallin poreux ayant un paramètre de diffusion supérieur à 15 s⁻¹ a subit un prétraitement à la vapeur à une température supérieure à 1000°C afin d'ajuster le paramètre de diffusion dudit matériau à une valeur de 0,1 à 15 s⁻¹ et l'étuvage réduit le volume des pores du catalyseur lorsqu'il est mesuré par adsorption de n-hexane à une température de 90°C et une pression de n-hexane de 10 kPa (75 torr) non inférieure à 50 % de celui du catalyseur avant ledit traitement à la vapeur.

2. Procédé selon la revendication 1, dans lequel ledit paramètre de diffusion dudit matériau cristallin poreux est de 0,5 à 10 s⁻¹.

3. Procédé selon la revendication 1, dans lequel ledit matériau cristallin poreux a subi un prétraitement à la vapeur pendant une période comprise entre 10 minutes et 100 heures.

4. Procédé selon la revendication 1, dans lequel le catalyseur contient au moins un modificateur d'oxyde choisi parmi des oxydes des éléments des groupes IIA, IIIA, IIIB, IVA, IVB, VA et VIA du Tableau Périodique.

5. Procédé selon la revendication 1, dans lequel le catalyseur contient au moins un modificateur d'oxyde choisi parmi des oxydes de bore, de magnésium, de calcium, de lanthane et de phosphore.

6. Procédé selon la revendication 1, dans lequel le catalyseur contient de 0,05 à 20 % en poids du modificateur d'oxyde sur la base du modificateur élémentaire.

7. Procédé selon la revendication 1, dans lequel le catalyseur contient de 0,1 à 10 % en poids du modificateur d'oxyde sur la base du modificateur élémentaire.

8. Procédé selon la revendication 1, dans lequel le catalyseur a une dimension moyenne des particules de 20 à 200 micromètres

9. Procédé selon la revendication 1, dans lequel le matériau cristallin poreux est une zéolite de type aluminosilicate.

10. Procédé selon la revendication 9, dans lequel ladite zéolite est ZSM-5 ou ZSM-11.

11. Procédé selon la revendication 1, dans lequel lesdites conditions d'alkylation comprennent une température entre 500 et 700°C, une pression entre 100 et 7000 kPa (de 1 atmosphère à 1000 psig), une vitesse spatiale horaire en poids entre 0,5 et 1000 et un rapport molaire du toluène au méthanol d'au moins 0,2.

12. Procédé selon la revendication 1, dans lequel ladite alkylation est réalisée en présence d'hydrogène et/ou d'eau supplémentaire de sorte que le rapport molaire de l'hydrogène et/ou de l'eau au toluène + méthanol dans l'alimentation soit de 0,01 à 10.

13. Procédé pour la production d'un catalyseur destiné à une utilisation dans la production sélective de para-xylène en faisant réagir le toluène avec le méthanol, ledit procédé comprenant les étapes consistant à :
(a) commencer avec un matériau cristallin poreux ayant un paramètre de diffusion pour le 2,2-diméthylbutane supérieur à 15 s⁻¹ lorsqu'il est mesuré à une température de 120°C et une pression de 2,2-diméthylbutane de 8 kPa (60 torr) ; et
(b) mettre en contact le matériau de l'étape (a) avec de la vapeur à une température d'au moins 1000°C afin de réduire le paramètre de diffusion de celui-ci pour le 2,2-diméthylbutane à environ de 0,1 à 15 s⁻¹ lorsqu'il est mesuré à une température de 120°C et une pression de 2,2-diméthylbutane de 8 kPa (60 torr), le volume des micropores du matériau étuvé lorsqu'il est mesuré par adsorption de n-hexane à une température de 90°C et une pression de n-hexane de 10 kPa (75 torr) étant au moins 50 % de celui du catalyseur avant ledit traitement à la vapeur.

14. Procédé selon la revendication 13, dans lequel le matériau cristallin poreux est combiné avec une source d'au moins un modificateur d'oxyde choisi parmi des oxydes des éléments des groupes IIA, IIIA, IIIB, IVA, IVB, VA et VIA du Tableau Périodique avant l'étape (b).

15. Procédé selon la revendication 13, dans lequel le matériau cristallin poreux est combiné avec une source d'au moins un modificateur d'oxyde choisi parmi des oxydes de bore, de magnésium, de calcium, de lanthane et de phosphore avant l'étape (b).

16. Procédé selon la revendication 13, dans lequel le matériau cristallin poreux utilisé dans l'étape (a) est une zéolite de type aluminosilicate ayant un indice de contrainte de 1 à 12.

17. Procédé selon la revendication 16, dans lequel la zéolite a un rapport molaire de la silice à l'alumine d'au moins 250.

18. Procédé selon la revendication 13, dans lequel l'étape (b) est réalisée à une température d'au moins 1000°C pendant une période de 10 minutes à 100 heures.
